(19) **Europäisches Patentamt**
**European Patent Office**
**Office européen des brevets**

(11) **EP 2 302 606 B1**

(12) **EUROPÄISCHE PATENTSCHRIFT**

(45) Veröffentlichungstag und Bekanntmachung des Hinweises auf die Patenterteilung:
**05.06.2013 Patentblatt 2013/23**

(51) Int Cl.:
*G08B 21/12* (2006.01)    *G08B 21/14* (2006.01)
*G08B 29/18* (2006.01)    *A61B 5/00* (2006.01)

(21) Anmeldenummer: **09171128.3**

(22) Anmeldetag: **23.09.2009**

(54) **Verfahren zur Alarmgenerierung, Steuereinrichtung und Vorrichtung zum Ausführen des Verfahrens**

Method for alarm generation, control device and device for carrying out the method

Procédé de génération d'alarme, dispositif de commande et dispositif d'exécution du procédé

(84) Benannte Vertragsstaaten:
**AT BE BG CH CY CZ DE DK EE ES FI FR GB GR HR HU IE IS IT LI LT LU LV MC MK MT NL NO PL PT RO SE SI SK SM TR**

(43) Veröffentlichungstag der Anmeldung:
**30.03.2011 Patentblatt 2011/13**

(73) Patentinhaber: **Dräger Medical GmbH**
**23558 Lübeck (DE)**

(72) Erfinder:
• **Franz, Frank**
  **23562 Lübeck (DE)**

• **Imhoff, Michael, Dr.**
  **44229 Dortmund (DE)**
• **Göpfert, Matthias, Dr.**
  **22529 Hamburg (DE)**

(74) Vertreter: **UEXKÜLL & STOLBERG**
**Patentanwälte**
**Beselerstrasse 4**
**22607 Hamburg (DE)**

(56) Entgegenhaltungen:
**EP-A- 0 540 144     DE-A1- 19 946 980**
**US-A1- 2008 208 026**

EP 2 302 606 B1

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

**Beschreibung**

Die vorliegende Erfindung betrifft ein Verfahren zur Alarmgenerierung gemäß dem Oberbegriff des Anspruchs 1. Sie betrifft ferner eine Steuereinrichtung gemäß Anspruch 7, eine Vorrichtung zur Alarmgenerierung gemäß Anspruch 8, eine Behandlungsvorrichtung gemäß Anspruch 12, ein digitales Speichermedium gemäß Anspruch 13, ein Computer-Programm-Produkt gemäß Anspruch 14 sowie ein Computer-Programm gemäß Anspruch 15.

Die Überwachung von Messwerten oder Parametern und die Generierung von Alarmen in kritischen Situationen ist Standard.

Eine Aufgabe der vorliegenden Erfindung ist, ein weiteres Verfahren zur Alarmgenerierung vorzuschlagen, falls ein überwachter Parameter von vorgegebenen ("normalen") Werten oder Wertebereichen abweicht.

U.S. 2008/0208026 A1 beschreibt einen Glukosemonitor, der die Glukosekonzentration im Blut mit einer variablen Wiederholrate misst, wobei bei einem sich langsam verändernden Glukosespiegel eine relativ niedrige Wiederholrate mit 1 Messung pro 10 Minuten und bei schnelleren Anstieg oder Abfall des Glukosespiegels eine höhere Wiederholrate von zum Beispiel 1 Messung pro Minute angewendet wird. Ferner arbeitet das Verfahren mit verschiedenen Schwellenwerten, um die Anpassung der Wiederholrate der Messungen zu berechnen.

Diese Aufgabe der vorliegenden Erfindung wird durch ein Verfahren zur Alarmgenerierung mit den Merkmalen des Anspruchs 1 gelöst.

Vorteilhafte Weiterentwicklungen sind Gegenstand von Unteransprüchen.

Bei allen folgenden Ausführungen ist der Gebrauch des Ausdrucks "kann sein" bzw. "kann haben" usw. synonym zu "ist vorzugsweise" bzw. "hat vorzugsweise" usw. zu verstehen.

Die Begriffe "Wert" und "Wertebereich" werden im Folgenden synonym oder austauschbar verwendet.

Die Verwendung der Singularform im Zusammenhang mit den Bezeichnungen Parameter, Messwert, Wert oder Wertebereich ist gleichwertig zur Pluralform zu verstehen und umgekehrt, da es sich im Rahmen der vorliegenden Erfindung jeweils um das Vorliegen oder Erfassen einer einzelnen Größe oder einer Vielzahl dieser Größen handeln kann.

Der Begriff "Parameter" oder "Messwert", wie er hierin verwendet wird, bezeichnet eine Größe bzw. Messgröße oder Kenngröße oder eine Ausprägung desselben, welche zum Treffen einer Aussage über einen zu überwachenden Zustand oder eine Änderung desselben herangezogen werden soll. Die Größe kann eine charakteristische Größe sein. Sie kann eine von einer Messgröße abgeleitete Größe sein. Es können mehrere Parameter bzw. Messwerte oder eine oder mehrere davon abgeleitete Größen überwacht werden. Es können mit dem erfindungsgemäßen Verfahren auch Abhängigkeiten einzelner Parameter oder Messwerte voneinander überwacht werden. Beispiele für mittels des erfindungsgemäßen Verfahrens überwachte Parameter oder Messwerte schließen physiologische Parameter, insbesondere Vitalparameter wie Herzschlag, Atmung, Temperatur usw., eines Patienten ein. Ebenso schließen sie pathologische Signalmuster, z.B. Arrhythmieanalyse im EKG ein.

Ferner schließen die überwachten Messgrößen Messwerte analytischer Verfahren, der Umweltanalytik oder Messgrößen von Prozessüberwachungen und dergleichen ein. Obwohl die vorliegende Erfindung im Folgenden im Wesentlichen unter Bezugnahme auf physiologische oder pathophysiologische Parameter oder Variablen beschrieben wird, ist die Erfindung nicht auf eine Anwendung im Bereich medizinischer oder medizintechnischer Verfahren beschränkt. Vielmehr ist die Erfindung für alle Ausführungsformen oder Ausgestaltungen anwendbar, in denen Parameter bzw. Messwerte überwacht werden.

Der Begriff "Abweichen", wie er hierin verwendet wird, bezeichnet das Über- oder Unterschreiten des Parameters über bzw. unter einen vorgegeben Wert oder Wertebereich. Dabei kann das Abweichen als der Beginn des Über- oder Unterschreitens zu verstehen sein. Es kann aber auch als der Zeitpunkt verstanden werden, an welchem der erste Ausschlag des nach einem über eine Zeitdauer hinweg diesseits des vorgegeben Werts verlaufen Parameters sein Maximum jenseits des Werts annimmt, oder Kombinationen hiervon. Außerdem kann das Abweichen auch als Veränderung der Dynamik des Wertverlaufs definiert werden. Beispielsweise kann dies in Form einer signifikanten Änderung im Anstieg oder in der Varianz des Werteverlaufs ausgeprägt sein. Weitere, dem Fachmann bekannte Möglichkeiten, das Abweichen zu definieren, sind ebenfalls von der Erfindung umfasst.

Unter "Werten oder Wertebereichen" (im Folgenden auch als normale Werte oder Wertebereiche bezeichnet) können diejenigen Werte oder Wertebereiche verstanden werden, die unter üblichen Bedingungen herrschen oder als Normbereich oder vom Fachmann als nicht alarmpflichtiger oder -würdiger Bereich verstanden werden. Derartige Werte oder Wertebereiche können willkürlich vorgegebene Werte oder Wertebereiche sein. Sie können auf (bereits vor der konkreten Überwachung) erfassten Messwerten oder Erfahrungswerten beruhen. Derartige Werte oder Wertebereiche können sich auf eine breite Erfahrungsbasis stützen; sie können allerdings auch fallspezifisch (z.B. für einen bestimmten Patienten) ermittelt und als normal festgelegt worden sein.

Befindet sich der zu überwachende Parameter im Bereich normaler Werte bzw. innerhalb eines normalen Wertebereichs, so besteht definitionsgemäß kein Anlass zum Handeln bzw. Eingreifen.

Ein "Patient" im Sinn der vorliegenden Erfindung kann ein Mensch oder ein Tier sein. Der Patient kann krank oder gesund sein. Der Patient kann einer medizinischen Behandlung bedürfen oder nicht.

Ein "Erfassen oder Bestimmen des Parameters" kann ein Messen des zu überwachenden Parameters oder Messwerts selbst beinhalten. Ebenso ist möglich, eine mit dem Parameter oder Messwert assoziierte Messgröße zu messen und diese in den gewünschten, zu überwachenden Parameter umzuwandeln.

Der Parameter kann kontinuierlich oder in diskreten Zeitintervallen bzw. zu bestimmten Zeitpunkten bzw. in bestimmten Zeiträumen erfasst werden.

Um einerseits ein Abweichen des Parameters von vorgegebenen Werten oder Wertebereichen bestimmen zu können, andererseits aber die Wahrscheinlichkeit möglicher Fehlalarme niedrig zu halten, ist erfindungsgemäß vorgesehen, den Parameter über ein zeitlich begrenztes Verifikationsintervall zu erfassen. Nimmt der überwachte Parameter innerhalb des Verifikationsintervalls wieder normale Werte an, d.h. liegt innerhalb des Verifikationsintervall keine Über- oder Unterschreitung des Schwellenwerts und/oder kein Abweichen des Parameters von vorgegebenen Werten oder Wertebereichen mehr vor, so wird dieses kurzzeitige Abweichen als Ausreißer verstanden, der zu keinen Konsequenzen führen soll. In Folge dieses Verständnisses wird in einer erfindungsgemäßen Ausgestaltung in einem solchen Fall kein Alarm ausgelöst.

Unter dem Begriff "zeitlich begrenztes Verifikationsintervall", wie er hierin verwendet wird, kann ein sich an den Zeitpunkt des Abweichens des Parameters anschließendes oder zu diesem Zeitpunkt beginnendes Zeitintervall verstanden werden. Erfindungsgemäß ist vorgesehen, die Länge oder Zeitdauer des Verifikationszeitintervalls in Abhängigkeit vom Ausmaß des Abweichens des Parameters von den vorgegebenen Werten oder Wertebereichen festzulegen bzw. variierbar oder variabel einzustellen.

In einer Ausführungsform des erfindungsgemäßen Verfahrens werden ein erster und ein zweiter Schwellenwert oder Schwellenwertbereich festgelegt. Der erste Schwellenwert kann beispielsweise ein oberer Schwellenwert oder Schwellenwertbereich sein, welcher einem maximalen oberen Gefährdungsgrad für den Patienten oder den überwachten Prozess entspricht. Analog hierzu kann der erste Schwellenwert natürlich ebenso - auch ergänzend - ein unterer Schwellenwert oder Schwellenwertbereich sein. Die Begriffe "Schwellenwert" oder "Schwellenwertbereich", wie sie hierin verwendet werden, bezeichnen einen Grenzwert oder Grenzwertbereich des Parameters bzw. Messwerts, bei dessen Über- oder Unterschreiten eine Feststellung getroffen oder eine Maßnahme eingeleitet oder beendet wird.

Ein Gefährdungsgrad - oder eine Kritikalität - kann dabei die Schwere oder Konsequenz des Abweichens des Parameters von normalen Werten oder Wertebereichen für den überwachten Prozess, vor allem natürlich für einen überwachten Patienten, widerspiegeln.

In einer weiteren Ausführungsform des erfindungsgemäßen Verfahrens werden zusätzliche mittlere Schwellenwerte oder Schwellenwertbereiche zwischen dem oberen Schwellenwert oder Schwellenwertbereich und dem vorgegebenen Wert oder Wertebereich - und/oder zwischen dem unteren Schwellenwert oder Schwellenwertbereich und dem vorgegebenen Wert oder Wertebereich - bestimmt bzw. festgelegt, um verschiedene Gefährdungsgrade darzustellen.

Der Begriff "mittlerer Schwellenwert oder Schwellenwertbereich" bezeichnet einen von den normalen Werten oder Wertebereichen abweichenden Wert oder Wertebereich mittleren Gefährdungspotentials.

Es können jeweils ein oder mehrere solcher mittlerer Schwellenwerte oder Schwellenwertbereiche (im Folgenden zusammenfassend auch nur als Schwellenwert bezeichnet) festgelegt bzw. bestimmten werden. Die mittleren Schwellenwerte können in gleichverteilten Abständen bzw. mit festen Werteabständen zwischen dem oberen oder unteren Grenzwert und dem normalen Wert oder Wertebereich angegeben werden. Die Abstände der mittleren Schwellenwerte voneinander oder vom oberen und/oder unteren Grenzwert können willkürlich oder in Abhängigkeit des sich verändernden Gefährdungspotentials bestimmt werden. Letzteres kann z.B. linear oder nicht linear, beispielsweise exponentiell, ansteigend zunehmen. Die Abstände zwischen den Schwellenwerten können daher ungleichmäßig sein.

Jeder Wert, jeder Schwellenwert, insbesondere aber die zusätzlichen mittleren Schwellenwerte, können auf Erfahrungswerten beruhen und/oder anhand von Daten einer vor Überwachungsbeginn vorliegenden Wissensbasis bestimmt werden.

Diese Werte können jedoch auch aufgrund von während der Überwachung ermittelten Überwachungswerten festgelegt werden. Die Festlegung kann automatisch erfolgen. Die Festlegung kann wiederholt erfolgen. Insbesondere kann eine Korrektur der Festlegung erfolgen.

Der Begriff "Erfahrungswert", wie er hierin verwendet wird, bezeichnet einen Wert, der sich aus einer persönlichen und/oder kollektiven Erfahrung ableiten lässt, beispielsweise von Klinikpersonal als typisch erkannt wurde. Ein Erfahrungswert kann ein für eine Zielgruppe von Patienten oder für einen konkreten Patienten zutreffender Wert sein. Beispielsweise geht man üblicherweise davon aus, dass sich die Herzschlagfrequenz oder Herzfrequenz (HF) eines gesunden Neugeborenen in Ruhe von jener eines gesunden 70-jährigen unterscheidet. Liegen bestimmte physische oder psychische Zustände vor, so können die Erfahrungswerte hiervon wiederum abweichen.

Der Begriff "Wissensbasis", wie er hierin verwendet wird, bezeichnet eine Wissensdatenbank, welche der Sammlung von Informationen wie beispielsweise Parametern, Messwerten oder Verläufen, d.h. Änderungen der Parameter oder Messwerte über die Zeit, dient. Die Wissensbasis kann parameter- und/oder patientengruppenspezifische Werte für einen bestimmten Parameter oder Messwert oder eine Ausprägung desselben aufweisen bzw. beinhalten.

Die Wissensbasis kann Erfahrungswerte wie oben beschrieben umfassen.

Zur automatischen Bestimmung der mittleren Schwellenwerte anhand der in der Wissensbasis hinterlegten Daten kann eine Steuereinrichtung, wie ein Mikroprozessor, verwendet werden.

Schwellenwerte können beim erfindungsgemäßen Verfahren auf verschiedene Weise automatisch eingestellt werden.

In einer Ausführungsform des erfindungsgemäßen Verfahrens wird ein erster Schwellenwert als niedrigster Gefährdungsgrad eingestellt und ein zweiter Schwellenwert als höchster Gefährdungsgrad anhand von Daten der Wissensbasis bestimmt.

Der erste Schwellenwert kann ein von einem Nutzer (Ärzte, Pflegepersonal usw.) angegebener Wert oder Wertebereich sein. Er kann auf Erfahrungswerten beruhen. Er kann das Messergebnis einer aktuellen Messung an einem Patienten widerspiegeln. Er kann ein willkürlich festgelegter Wert sein.

Der Nutzer kann Werte mittels einer dazu vorgesehenen Schnittstelle, wie z.B. über ein Tastatur, in eine Steuereinrichtung, wie einen Mikroprozessor, eingeben. Der zweite Schwellenwert kann ein parameter- und/oder patientengruppenspezifischer sein.

Der durch den zweiten Schwellenwert vorgegebene höchste Gefährdungsgrad kann ein Notfallschwellenwert sein. Der Begriff "Notfallschwellenwert" bezeichnet einen Wert eines Parameters oder Messwerts, bei dessen Erreichen unverzüglich Notfallmaßnahmen zu treffen sind.

Basierend auf dem vom Nutzer eingestellten ersten Schwellenwert und einem in der Wissensbasis hinterlegten bzw. aus dieser abrufbaren parameter- und/oder patientengruppenspezifischen Notfallschwellenwert als zweitem Schwellenwert, bestimmt oder berechnet der Mikroprozessor, zum Beispiel durch Interpolation, n (dies kann eine natürliche ganze Zahl sein) Zwischenstellen bzw. mittlere Schwellenwerte.

In einer weiteren Ausführungsform wird ein erster Schwellenwert von einem Nutzer als höchster Gefährdungsgrad eingestellt und mittlere Schwellenwerte und ein unterer Schwellenwert werden anhand von Daten der Wissensbasis bestimmt oder errechnet.

Basierend auf dem vom Nutzer eingestellten Schwellenwert und ggf. anhand von Informationen über den zu überwachenden Parameter, wie beispielsweise Mittelwert und/oder Varianz innerhalb der letzten 5 Minuten, können vom Mikroprozessor automatisch n patientenspezifische Schwellen bestimmt werden. In einem Fall, in dem keine Historie zu dem zu überwachenden Parameter vorliegt, können zunächst in der Wissensbasis hinterlegte Normwerte bzw. normale Werte oder Wertebereiche zur Festlegung einer, mehrerer oder aller Schwellen oder Grenzwerte herangezogen werden.

Der durch den Nutzer eingegebene Schwellenwert kann dem höchsten Gefährdungsgrad entsprechen, die weiteren Schwellenwerte oder Schwellenwertbereiche (d.h. die mittleren Schwellenwerte und der untere Schwellenwert) niedrigeren Gefährdungsgraden.

In einer weiteren Ausführungsform wird ein spezifisches Verifikationsintervalls einem spezifischen Gefährdungsgrad zugeordnet.

Der Begriff "spezifisches Verifikationsintervall" bezeichnet ein Verifikationsintervall mit einer bestimmten, zeitlich begrenzten Verifikationsintervalllänge. Die Länge des Verifikationsintervalls nimmt dabei vorzugsweise mit steigendem Gefährdungsgrad ab. Die definierte Verzögerungsintervalllänge für den niedrigsten Gefährdungsgrad entspricht vorzugsweise der maximal möglichen Alarmverzögerung bei einer geringfügigen Überschreitung des entsprechenden Schwellenwerts oder Schwellenwertbereichs. Bei starken Abweichungen des Parameters von den vorgegebenen normalen Werten oder Wertebereichen kann auf diese Weise hingegen sehr schnell alarmiert werden. Die Zuordnung der spezifischen Verifikationsintervalllänge zu einem spezifischen Gefährdungsgrad kann anhand von Daten der Wissensbasis erfolgen. Die Länge des Verifikationsintervalls, welchem ein bestimmter Gefährdungsgrad zugeordnet wird, kann ein parameter- und/oder patientengruppenspezifischer Wert sein. Der Wert kann entsprechend in der Wissensbasis des Systems, beispielsweise in einem Datenspeicher abgelegt oder hinterlegt sein.

In einer Ausführungsform des erfindungsgemäßen Verfahrens ist vorgesehen, den Gefährdungsgrad anhand von Daten des Parameters oder Messwerts einer Mehrheit von Patienten oder Probanden zu bestimmen. Die generalisierten Daten können dann auf einen individuellen Patienten übertragen werden.

Alternativ dazu kann die Zuordnung situationsspezifisch anhand der Stabilität oder Instabilität und/oder "Kritikalität" eines Patientenzustands erfolgen.

Die Werte für die Verifikationsintervalllänge können situationsspezifisch skaliert werden. Beispielsweise kann eine Verkürzung der Verifikationsintervalle bei instabilen Patienten vorteilhaft zu empfindlicheren - im Sinne von frühzeitigeren Alarmen - führen.

Zur Automatisierung einer Skalierung ist es möglich, in vielen Bereichen - wie der Medizin - als solche benannte "Scores" einzusetzen, die eine Aussage über die Stabilität / Kritikalität z.B. des Zustands des Patienten liefern.

Hierbei kann es vorteilhaft möglich sein, die Verifikationsintervalllänge individuell (auch) an das Erfordernis eines ganz konkreten Patienten anzupassen. Auf diese Weise kann es wiederum vorteilhaft möglich sein, die Überwachung eines Patienten weiter zu optimieren.

In einer weiteren Ausführungsform des erfindungsgemäßen Verfahrens ist vorgesehen, wenigstens ein Zeitintervall, in welchem der Parameter um mehr als einen ersten Schwellenwert oder einen Schwellenwertbereich von den vorgegebenen normalen Werten oder Wertebereichen abweicht, aufzuzeichnen.

Das Aufzeichnen des Zeitintervalls kann in geeigneter Weise automatisch oder automatisiert erfolgen. Geeignete Einrichtungen können hierzu vorgesehen sein.

Vorzugsweise werden Ergebnisse der Aufzeichnung in einer geeigneten Speichervorrichtung hinterlegt.

Die Dauer des Zeitintervalls der Abweichung kann in Zeiteinheiten gemessen und aufgezeichnet werden. Je nach Anwendung des Verfahrens können geeignete Zeiteinheiten z.B. $\mu$s, s, min oder h umfassen. Zum Erfassen und/oder Aufzeichnen der Zeiteinheiten kann ein Zähler eingesetzt werden.

Der Begriff "Zähler", wie er hierin verwendet wird, bezeichnet eine Einrichtung, welche zum Erfassen und ggf. Anzeigen der Dauer des Zeitintervalls geeignet ist. Der Zähler kann ein elektronischer Zähler sein. Er kann ein Mengenzählwerk sein, welches zum Zählen kontinuierlicher Größen ausgelegt ist. Je nach Erfordernis bzw. Genauigkeit und Situation kann der Zähler ausgelegt sein, Zeiteinheiten in ganzen Zeiteinheiten, wie beispielsweise 1 s, 2 s, 4 s, 10 s, 20 s, 50 s, oder in beliebigen Teil-Zeiteinheiten hiervon zu erfassen. Der Zähler kann ausgestaltet sein, um zwischen verschiedenen Zeiteinheiten wechseln zu können. Der Zähler kann in eine Steuereinrichtung integriert sein bzw. Teil einer solchen bilden. Er kann über geeignete Verbindungen, wie Leitungen, Schnittstellen und dergleichen, mit einer Steuereinrichtung verbindbar oder verbunden sein. Es kann ein einziger Zähler vorgesehen sein, um die Dauer des Zeitintervalls der insgesamt erfolgenden Abweichung des Parameters von den vorgegebenen normalen Werten oder Wertebereichen zu erfassen. Es können mehrere gleich ausgestaltete oder verschiedenartig ausgestaltete Zähler vorgesehen sein, um die Dauer des Zeitintervalls der Abweichung des Parameters von einem Wert zu einem weiteren, z.B. dem nächst höher oder tiefer liegenden Wert, zu erfassen. Beispielsweise kann ein erster Zähler dazu vorgesehen sein, die Dauer des Zeitintervalls der Abweichung des Parameters von den vorgegebenen normalen Werten oder Wertebereichen zu erfassen. Ein zweiter Zähler kann vorgesehen sein, um die Dauer des Zeitintervalls der Abweichung des Parameters von einem mittleren Schwellenwert zu erfassen. Ein dritter Zähler kann vorgesehen sein, um die Dauer des Zeitintervalls der Abweichung des Parameters von einem oberen oder unteren Schwellenwert oder Schwellenwertbereich, ggf. des Notfallschwellenwerts, zu erfassen. Weitere Zähler sind möglich.

In einer weiteren Ausführungsform des erfindungsgemäßen Verfahrens bleibt der Zähler solange auf Null gesetzt, bis der Parameter um mehr als einen ersten Schwellenwert von den vorgegebenen normalen Werten abweicht.

In einer solchen Ausgestaltung des erfindungsgemäßen Verfahrens ist entsprechend vorgesehen, dass der Zähler erst zu zählen beginnt, wenn der Parameter von den normalen Werten abweicht. Wenn mehrere Zähler vorgesehen sind, kann es entsprechend möglich sein, dass ein erster Zähler, welcher die Dauer des Zeitintervalls der Abweichung des Parameters von den vorgegebenen normalen Werten oder Wertebereichen erfasst, bereits zu zählen beginnt, während ein zweiter oder dritter Zähler noch auf Null gesetzt ist.

In einer weiteren Ausführungsform des erfindungsgemäßen Verfahrens ist vorgesehen, ein Alarmsignal auszugeben, wenn der Parameter über die zeitliche Begrenzung des Verifikationsintervalls um mehr als einen ersten Schwellenwert von den vorgegebenen normalen Werten abweicht. Genauer gesagt, bedeutet dies, dass die Dauer des Zeitintervalls der Abweichung größer als die zeitliche Begrenzung des Verifikationsintervalls ist.

In einer weiteren Ausführungsform des erfindungsgemäßen Verfahrens wird der Zähler (um z.B. eine Zeiteinheit) dekrementiert, wenn der Parameter eine Zeiteinheit lang auf einen Wert unterhalb eines für den Zähler relevanten Schwellenwertes absinkt und der Wert des Zählers größer Null ist. Auf diese Weise kann die Länge bzw. Dauer des Zeitintervalls der Abweichung des Parameters verringert werden. Eine solche Vorgehensweise kann insbesondere bei um einen bestimmten Werte oder Wertebereich alternierenden bzw. schwankenden Parametern von Vorteil sein. Eine derartige Dekrementfunktion kann vorteilhaft dazu beitragen, das zügige Alarmieren auch bei alternierenden bzw. schwankenden Messwerten zu gewährleisten. Wenn der Parameter erneut über den Schwellenwert oder Schwellenwertbereich (auch diese beiden Begriffe werden hier zusammenfassen aus Schwellenwert bezeichnet) hinaus abweicht, beginnt der Zähler zu laufen und es werden die Zeiteinheiten, die der Dauer des erneuten Abweichens des Parameters entsprechen, zu dem aktuellen Stand des Zählers addiert. Auf diese Weise kann vorteilhaft sichergestellt werden, dass ein kritisches Abweichen des Parameters trotz alternierendem bzw. schwankendem Verlauf des Parameters sicher erfasst werden kann.

Solange die vom Zähler gezählten Zeiteinheiten der Abweichung des Parameters kleiner als das Verifikationsintervall sind, wird kein Alarmsignal ausgelöst, da der Verlauf des Parameters bzw. seine Abweichung von den normalen Werten oder Wertebereichen als unkritisch angesehen werden kann. Wenn die vom Zähler gezählten Zeiteinheiten hingegen die Zeitdauer des in Abhängigkeit des Gefährdungsgrades bestimmten Verifikationsintervalls übersteigen, wird in dieser Ausführungsform ein Alarmsignal ausgegeben.

Das erfindungsgemäße Verfahren ist auf die Anpassung der Verifikationsintervalllänge an den durch das Ausmaß der

Schwellenwertüberschreitung bestimmten Gefährdungsgrad gerichtet. Dabei werden vorteilhaft nicht nur die bloße Schwellenwertverletzung über einen kurzen Zeitraum, sondern ebenso Betrag und Dauer der Grenzwertverletzung berücksichtigt.

Die erfindungsgemäße Aufgabe wird gleichermaßen durch eine erfindungsgemäße Steuereinrichtung gemäß Anspruch 6 gelöst. Alle Vorteile des erfindungsgemäßen Verfahrens lassen sich ungeschmälert auch mit der erfindungsgemäßen Steuereinrichtung erreichen. Die erfindungsgemäße Steuereinrichtung ist dazu konfiguriert oder ausgelegt und/oder vorgesehen, das erfindungsgemäße Verfahren ganz oder in Teilschritten desselben auszuführen. Die erfindungsgemäße Steuereinrichtung kann eine computergestützte Steuereinrichtung, wie ein Mikroprozessor, eine CPU, und dergleichen sein.

Die Steuereinrichtung kann in einen Rechner oder Computer integriert oder integrierbar sein. Die Steuereinrichtung kann geeignete Einrichtungen zur Kommunikation mit anderen Einrichtungen, wie Benutzerschnittstellen und dergleichen, aufweisen. Die erfindungsgemäße Steuereinrichtung ist vorzugsweise dazu ausgelegt, die zu überwachenden, erfassten Parameter oder Messwerte zu verarbeiten. Die Steuereinrichtung kann dazu ausgelegt sein, Berechnungen vorzunehmen und/oder Daten an externen Schnittstellen zur Verfügung zu stellen. Zum Abspeichern oder Hinterlegen von Ergebnissen kann die Steuereinrichtung mit wenigstens einer Speichereinrichtung oder Speicher-/Leseeinrichtung verbindbar oder verbunden sein.

Die Steuereinrichtung kann dazu vorgesehen sein, die erfassten Parameter oder Messwerte auf der Benutzerschnittstelle grafisch und/oder numerisch zur Anzeige zu bringen. Zu diesem Zweck kann die Steuereinrichtung mit wenigstens einer Eingabeeinrichtung zum Eingeben der vorgegebenen normalen Werte verbindbar oder verbunden sein. Die Steuereinrichtung kann Eingaben eines Nutzers, wie beispielsweise vorgegebene Werte, von der Benutzerschnittstelle entgegennehmen, auswerten und/oder die erhaltenen Ergebnisse in entsprechendes Geräteverhalten umsetzen. Ferner kann die Steuereinrichtung dazu vorgesehen sein, den Alarmstatus aller zu überwachenden Parameter vorzugsweise fortwährend oder stetig zu überprüfen und zu aktualisieren.

Um eine Dauer des Zeitintervalls des Abweichens eines Parameters um mehr als einen ersten Schwellenwert von vorgegebenen normalen Werten zu erfassen, kann die Steuereinrichtung vorzugsweise wenigstens einen Zähler aufweisen. Die Steuereinrichtung kann ferner dazu vorgesehen sein, ein Alarmsignal bei Bedarf, d.h. bei Auf- bzw. Eintreten eines kritischen Zustands, zu generieren bzw. zu veranlassen.

Die erfindungsgemäße Aufgabe wird gleichermaßen durch eine erfindungsgemäße Vorrichtung zur Alarmgenerierung gemäß Anspruch 11 gelöst. Alle Vorteile des erfindungsgemäßen Verfahrens lassen sich ungeschmälert auch mit der erfindungsgemäßen Vorrichtung erreichen. Die erfindungsgemäße Vorrichtung zur Alarmgenerierung weist wenigstens eine Erfassungseinrichtung zum Erfassen oder Bestimmen der aktuellen Ausprägung eines überwachten Parameters, wenigstens eine Vergleichseinrichtung zum Vergleichen des erfassten (aktuellen) Parameters mit vorgegebenen normalen Werten oder Wertebereichen, und wenigstens eine erfindungsgemäße Steuereinrichtung auf. Der Begriff "Erfassungseinrichtung", wie er hierin verwendet wird, bezeichnet eine Einrichtung, die zum Erfassen oder Messen eines Parameters oder eines Messwerts geeignet ist.

Die Erfassungseinrichtung kann eine Mess- und Vorverarbeitungseinheit oder - einrichtung sein. Die Erfassungseinrichtung kann ein Sensor sein oder mit einem oder mehreren Sensoren verbunden sein. Je nach Verwendungszweck der erfindungsgemäßen Vorrichtung schließen geeignete Sensoren eine breite Vielfalt an Ausgestaltungen ein, darunter Sensoren zur Erfassung biologischer und/oder physiologischer Eigenschaften, wie Biosignale (EKG, EEG, Temperatur) vom Patienten, Sensoren zur Erfassung chemischer und/oder physikalischer Eigenschaften, wie Temperatursensoren, Druckmesssensoren, Sensoren zur Messung von Konzentrationen oder Konzentrationsänderungen und dergleichen. Die von der Erfassungseinrichtung erfassten Parameter oder Messwerte können bei Bedarf in weiterverarbeitbare Größen (meist elektrische Signale) umgeformt werden. Geeignete Vorverarbeitungseinrichtungen, wie Verstärker, A/D-Wandler, Filter und dergleichen können vorgesehen und/oder in die Erfassungseinrichtung integriert sein. Aus den aufbereiteten bzw. vorverarbeiteten Parametern oder Messwerten können weitere Parameter abgeleitet werden, wie z.B. die Herzfrequenz aus dem EKG.

Der Begriff "Vergleichseinrichtung", wie er hierin verwendet wird, bezeichnet eine Einrichtung, welche dazu vorgesehen und ausgelegt ist, um den aktuell erfassten Parameter oder Messwert mit den vorgegebenen normalen Werten oder Wertebereichen zu vergleichen. Beispiele für geeignete Vergleichseinrichtungen schließen Einrichtungen ein, welche eine Subtraktion des aktuell erfassten Parameters oder Messwerts vom vorgegeben normalen Wert oder umgekehrt ausführen. Je nach der aus dem Subtraktionsvorgang erhaltenen Differenz kann auf eine Schwellenwertverletzung durch den zu überwachenden Parameter oder Messwert geschlossen werden. Das Ausmaß der Schwellenwertverletzung über die Dauer des Verifikationsintervalls hinaus kann zur Auslösung eines Alarmsignals führen.

In einer weiteren Ausführungsform weist die erfindungsgemäße Vorrichtung wenigstens eine Alarmeinrichtung zum Ausgeben eines Alarmsignals auf.

Die Ausgabe des Alarmsignals kann mittels akustischer und/oder visueller Darstellung erfolgen. Das Alarmsignal kann erfindungsgemäß ergänzend oder alternativ (d.h. ausschließlich) in einem Signal zum Beenden oder Beginnen eines Vorganges bestehen. So kann die Gabe eines Medikaments in einem erkannten Alarmzustand gestoppt oder eingesetzt werden. Der Begriff "Alarm" und "Alarmzustand" wie hier verwendet ist erfindungsgemäß daher nicht auf eine optische oder akustische Mitteilung beschränkt.

In einer weiteren Ausführungsform weist die erfindungsgemäße Vorrichtung wenigstens eine Speichereinrichtung oder Speicher-/Leseeinrichtung zum Abspeichern oder Hinterlegen von Ergebnissen auf. Die Begriffe "Speichereinrichtung" oder "Speicher-/Leseeinrichtung" bezeichnen einen Datenspeicher oder ein Speichermedium, das üblicherweise der Speicherung und Sicherung von Daten bzw. Informationen dient. Die Steuereinrichtung kann in bestimmten Ausführungsformen lesend und schreibend auf die Speichereinrichtung oder Speicher-/Leseeinrichtung zugreifen. Der Zugriff der Steuereinrichtung auf die Speichereinrichtung kann mittels geeigneter Datenverbindungen und/oder Datenanschlüssen, wie Datenverbindungen unter Verwenden von Kabeln oder kabellosen Datenverbindungen, z.B. SATA, IR, Bluetooth, WLAN und dergleichen erfolgen. Die Speichereinrichtung oder Speicher-/Leseeinrichtung kann in manchen Ausführungsformen in die Steuereinrichtung integriert sein. Ebenso oder zusätzlich dazu ist es möglich, eine externe Speichereinrichtung oder Speicher-/Leseeinrichtung einzusetzen.

Beispiele für integrierte Speichereinrichtungen oder Speicher-/Leseeinrichtungen schließen RAM, ROM, EPROM und dergleichen ein. Beispiele für externe Speichereinrichtungen oder Speicher-/Leseeinrichtungen schließen Disketten, CD-ROMs, CDs, CD-RWs (rewritable CD), DVDs, Speicherchips, SD-Karten (SD Memory Card), Chipkarten, USB-Sticks und dergleichen ein.

Die Speichereinrichtungen oder Speicher-/Leseeinrichtungen können verschiedene Bereiche oder Partitionen zur Ablage von Konfigurationsdaten, Wissensbasen, Nutzereinstellungen, Statusvariablen und/oder Verlaufsinformationen aufweisen. In bestimmten Ausführungsformen der erfindungsgemäßen Vorrichtung weist die Speichereinrichtung oder Speicher-/Leseeinrichtung wenigstens eine Datenbank auf oder ist mit einer solchen koppelbar. In der Datenbank sind bevorzugt Daten einer Wissensbasis hinterlegt oder hinterlegbar.

In einer Weiterbildung weist die erfindungsgemäße Vorrichtung wenigstens eine Eingabeeinrichtung zum Eingeben der vorgegebenen Werte oder Wertebereiche auf. Beispiele für derartige Eingabeeinrichtungen schließen eine Tastatur, eine Maus, ein Touch-Pad, ein Mikrophon, einen Scanner, ein Kartenlesegerät und dergleichen ein.

In einer Weiterbildung weist die erfindungsgemäße Vorrichtung wenigstens eine Ausgabeeinrichtung zum Ausgeben von Ergebnissen der Steuereinrichtung auf. Die Ausgabe der Daten oder Informationen kann graphisch, z.B. in Form einer dreidimensionalen Abbildung, und/oder numerisch, z.B. in Form eines Zahlenwerts, erfolgen. Geeignete Ausgabeeinrichtungen schließen einen Bildschirm, einen PDA (personal digital assistant), einen Drucker oder Plotter und dergleichen ein.

Die erfindungsgemäße Aufgabe wird gleichermaßen durch eine erfindungsgemäße Behandlungsvorrichtung gemäß Anspruch 12 gelöst. Alle Vorteile des erfindungsgemäßen Verfahrens lassen sich ungeschmälert auch mit der erfindungsgemäßen Behandlungsvorrichtung erreichen. Die erfindungsgemäße Behandlungsvorrichtung weist wenigstens eine erfindungsgemäße Steuereinrichtung oder wenigstens eine erfindungsgemäße Vorrichtung zur Alarmgenerierung auf. Gemäß einer Ausführungsform kann die erfindungsgemäße Behandlungsvorrichtung zum Überwachen eines Zustands eines Patienten ausgelegt und vorgesehen sein. Beispielsweise kann die Behandlungsvorrichtung dazu ausgelegt sein, die Herzfrequenz eines Patienten zu überwachen. Die Behandlungsvorrichtung kann eine im Bereich der Akutmedizin eingesetzte Behandlungsvorrichtung wie ein Beatmungsgerät, ein Anästhesiegerät oder dergleichen sein.

Die erfindungsgemäße Aufgabe wird gleichermaßen durch ein erfindungsgemäßes digitales Speichermedium gemäß Anspruch 13 gelöst. Alle Vorteile des erfindungsgemäßen Verfahrens lassen sich ungeschmälert auch mit dem erfindungsgemäßen digitalen Speichermedium erreichen. Das digitale Speichermedium, welches insbesondere eine Diskette, eine CD oder eine DVD ist, weist bevorzugt elektrisch auslesbare Steuersignale auf, die so mit einem programmierbaren Computersystem zusammenwirken können, dass ein erfindungsgemäßes Verfahren ausgeführt wird.

Die erfindungsgemäße Aufgabe wird gleichermaßen durch ein erfindungsgemäßes Computer-Programm-Produkt gemäß Anspruch 14 gelöst. Alle Vorteile des erfindungsgemäßen Verfahrens lassen sich ungeschmälert auch mit dem erfindungsgemäßen Computer-Programm-Produkt erreichen. Das Computer-Programm-Produkt weist vorzugsweise einen auf einem maschinenlesbaren Träger gespeicherten Programmcode zur Durchführung des erfindungsgemäßen Verfahrens auf, wenn das Programm-Produkt auf einem Rechner oder Computer abläuft.

Der Begriff "maschinenlesbarer Träger", wie er hierin verwendet wird, bezeichnet einen Träger, der von Software und/oder Hardware interpretierbare Daten oder Informationen enthält. Der Träger kann ein Datenträger, wie eine Diskette, eine CD, DVD und dergleichen sein. Die erfindungsgemäße Aufgabe wird gleichermaßen durch ein erfindungsgemäßes Computer-Programm gemäß Anspruch 15 gelöst. Alle Vorteile des erfindungsgemäßen Verfahrens lassen sich ungeschmälert auch mit dem erfindungsgemäßen Computer-Programm erreichen. Das Computer-Programm weist einen Programmcode zur Durchführung des erfindungsgemäßen Verfahrens auf, wenn das Programm auf einem Rechner oder Computer abläuft.

Die vorliegende Erfindung stellt vorteilhaft ein Verfahren sowie geeignete Einrichtungen und Vorrichtungen bereit, mit welchem Alarmsignale in vorteilhafter Weise zuverlässig und nachvollziehbar generiert werden können, falls zu überwachende physiologische Variablen oder davon abgeleitete Parameter einen vorgegebenen Bereich verlassen.

Mittels der vorliegenden Erfindung kann dabei vorteilhaft sichergestellt werden, dass Alarmsignale nur bei signifikanter Verletzung von Schwellenwerten oder Schwellenwertbereichen auftreten. Auf diese Weise kann es vorteilhaft möglich sein, die Anzahl gegebener Fehlalarme wesentlich zu reduzieren. Durch die flexible Art der Bestimmung der notwendigen gefährdungsgradspezifischen Schwellenwerte oder Schwellenwertbereiche und Verzögerungsintervalle kann das Verfahren vorteilhaft auf verschiedene Parameter und Patientengruppen angewendet werden.

Das erfindungsgemäße Verfahren kann aufgrund der gefährdungsgradspezifisch festgelegten Verifikationsintervalle vorteilhaft dazu beitragen, diejenige Anzahl an Alarmen zu reduzieren oder zu eliminieren, die nicht mit einem klinischen relevanten und ggf. kritischen Zustand eines Patienten assoziiert sind.

Fehlalarme im medizinischen Kontext werden vorrangig durch Artefakte im gemessenen physiologischen Signal, welche durch Bewegung, Eingriffe des Personals oder andere externe Störungen hervorgerufen werden, verursacht. Das erfindungsgemäße Verfahren kann vorteilhaft auch hier dazu beitragen, derartige falsch-positive Alarmsignale zu vermeiden oder die Häufigkeit ihres Auftretens zu verringern. Dabei kann das erfindungsgemäße Verfahren vorteilhaft auf eine Vielzahl von Verfahren mit einer durch Grenzwertalarmierung verursachten, hohen Fehlalarmquote angewendet werden.

Da unter Verwenden des erfindungsgemäßen Verfahrens vorteilhaft signifikant weniger Fehlalarme produziert werden, können Nutzer adäquater auf Alarmsignale und die damit in Zusammenhang stehenden Alarmereignisse reagieren.

Die mittels des erfindungsgemäßen Verfahrens vorteilhaft bereitgestellte Alarmsicherheit bei ausschließlich tatsächlich kritischen Zuständen, kann sich vorteilhaft positiv auf die Erwartungshaltung der Nutzer auswirken: Die Nutzer können Vertrauen in die vom erfindungsgemäßen Verfahren generierten Alarme haben. Auf diese Weise kann ferner vorteilhaft die Patientensicherheit erhöht werden; Nutzer werden aufgrund der zuverlässigen Alarmereignisse adäquat reagieren.

Aufgrund der erfindungsgemäß vorteilhaft erzielbaren Reduzierung von Fehlalarmen kann das Lärm- und Stresslevel im Anwendungsbereich der Überwachungsgeräte vorteilhaft verringert werden. Die Erfindung kann vorteilhaft die mit einer hohen Anzahl an generierten Alarmen einhergehende Belastung durch Lärm, Stress und die zusätzliche Arbeitsbelastung verringern. Die kann z.B. einem Patienten zugute kommen.

Das erfindungsgemäße Verfahren weist vorteilhaft eine geringe Komplexität auf, so dass die Alarmgenerierung für einen Nutzer vorteilhaft nachvollziehbar und durchschaubar wird. Auf diese Weise können das Vertrauen- und die Akzeptanz eines Nutzers vorteilhaft gestärkt werden. Der vorliegenden Erfindung liegt vorteilhaft ein einfaches Bedienkonzept zugrunde, welches sich an vorhandenen Konzepten und Erfahrungswerten der Nutzer orientiert und mit wenig Interaktion seitens des Nutzers auskommen kann.

Ein weiterer Vorteil des erfindungsgemäßen Verfahrens kann in seiner generellen Anwendbarkeit auch auf verschiedene physiologische Variablen und unterschiedliche Patientengruppen liegen.

Ferner ist es vorteilhaft möglich, das erfindungsgemäße Verfahren in allen Bereichen anzuwenden, die des Erfassens und Überwachens von Parametern oder Messwerten und einer entsprechenden Ausgabe von Alarmsignalen in echten kritischen Zuständen bedürfen. Das erfindungsgemäße Verfahren stellt somit vorteilhaft eine Generalisierbarkeit bereit.

Da auf komplizierte Berechnungen verzichtet wird, ist das beschriebene Verfahren besonders Ressourcen schonend, die Auslegung der beschriebenen Vorrichtung kann somit mit leistungsschwächeren und dafür energieeffizienten Mikroprozessoren erfolgen. Die vorliegende Erfindung wird im Folgenden anhand der beigefügten Zeichnung, in welcher identische Bezugszeichen gleiche oder ähnliche Bauteile bezeichnen, exemplarisch erläutert. In den zum Teil stark vereinfachten Figuren gilt:

Fig. 1     zeigt schematisch eine erfindungsgemäße Vorrichtung;

Fig. 2     zeigt einen Graphen zur Bestimmung automatisch generierter Schwellenwerte oder Schwellenwertbereiche gemäß einer ersten Alternative des erfindungsgemäßen Verfahrens; und

Fig. 3     zeigt einen Graphen zur Bestimmung automatisch generierter Schwellenwerte oder Schwellenwertbereiche gemäß einer zweiten Alternative des erfindungsgemäßen Verfahrens.

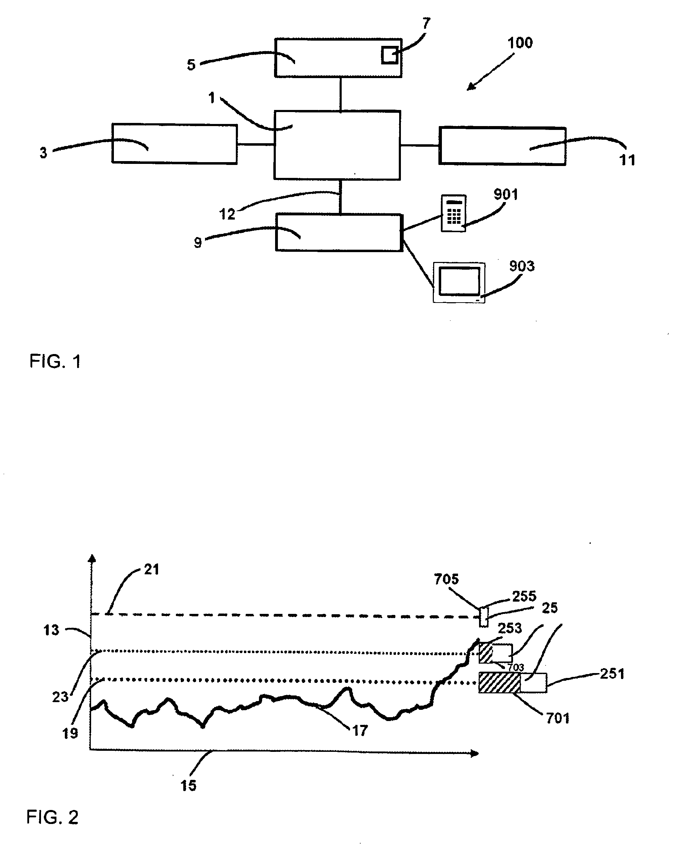

Fig. 1 zeigt schematisch eine Übersicht über Komponenten einer erfindungsgemäßen Vorrichtung 100 zur Alarmgenerierung.

Die Vorrichtung 100 zur Alarmgenerierung weist eine Steuereinrichtung 1, z.B. einen Mikroprozessor, auf.

Die Steuereinrichtung 1 ist mit einer Erfassungseinrichtung 3 verbunden. Die Erfassungseinrichtung 3 ist dazu vorgesehen, einen Parameter oder Messwert, welcher überwacht werden soll, zu erfassen oder zu bestimmen. Die Erfassungseinrichtung 3 kann ein Sensor sein.

Die Steuereinrichtung 1 ist mit einer Speicher-/Leseeinrichtung 5 verbunden. Die Speicher-/Leseeinrichtung 5 kann ein ROM oder RAM sein. Sie kann eine externe Speicher-/Leseeinrichtung, wie eine Diskette oder CD sein.

In der Steuereinrichtung 1 sind drei Zähler 7, d.h. für jede Schwelle einen, integriert. Die Steuereinrichtung 1 könnte allerdings auch jede andere Anzahl von Zählern aufweisen.

Die Zähler 7 sind dazu vorgesehen, die jeweilige Dauer des Zeitintervalls der Abweichung des Parameters von den vorgegebenen normalen Werten oder Wertebereichen über einen bestimmten Schwellenwert oder Schwellenwertbereich hinaus zu erfassen.

Die Steuereinrichtung 1 ist mit einer Benutzschnittstelle 9 verbunden. Die Benutzerschnittstelle 9 kann mit wenigstens einer Eingabeeinrichtung 901 zum Eingeben bzw. Hinterlegen von Parametern in die Speicher-/Leseeinrichtung 5, z.B. einer Tastatur, verbunden sein. Die Benutzerschnittstelle 9 kann mit wenigstens einer Ausgabeeinrichtung 903 zum Ausgeben bzw. Darstellen von Daten verbunden sein, z.B. einem Bildschirm.

Die Steuereinrichtung 1 ist ferner mit einer Alarmeinrichtung 11 verbunden. Die Alarmeinrichtung 11 ist dazu vorgesehen, einen Alarm, z.B. einen akustischen Alarm, auszugeben, falls der Parameter über die Länge eines festgelegten Verifikationsintervalls hinaus den normalen Wert oder Wertebereich über- oder unterschreitet.

Die einzelnen Komponenten der erfindungsgemäßen Vorrichtung 100 sind über eine Datenleitung 12 miteinander verbunden.

Zur Durchführung des erfindungsgemäßen Verfahrens wird beispielsweise von einem Nutzer über die Benutzerschnittstelle 9 für jeden zu überwachenden Parameter ein Schwellenwert oder Schwellenwertbereich festgelegt (bei der Überwachung eines Wertebereichs vorzugsweise ein oberer und ein unterer Schwellenwert oder Schwellenwertbereich).

Basierend auf diesem Schwellenwert oder Schwellenwertbereich und einer in der Speicher-/Leseeinrichtung 5 hinterlegten Wissensbasis können mittels der Steuereinrichtung 1 automatisch zusätzliche Schwellen bestimmt werden. Sie können verschiedene Gefährdungsgrade repräsentieren. Jedem dieser Schwellenwerte oder Schwellenwertbereiche wird eine entsprechende Verifikationsintervalllänge zugeordnet. Die Verifikationsintervalllänge nimmt dabei bevorzugt mit steigendem Gefährdungsgrad ab. So kann bei einer sehr starken Überschreitung der Schwellenwert oder Schwellenwertbereich mit dem kürzesten Verifikationsintervall verletzt und schnell ein Alarm gegeben werden, während bei einer geringfügigen Überschreitung die Ausgabe eines Alarms erst nach Ablauf eines längeren Verifikationsintervalls verzögert werden kann.

Während der Überwachung verwaltet die Steuereinrichtung 1 in der Speicher-/Leseeinrichtung 5 für jeden Schwellenwert oder Schwellenwertbereich wenigstens einen Zähler 7, der die Dauer der Schwellenwertverletzung repräsentiert.

Im Normalfall, d.h. wenn der zugehörige Schwellenwert oder Schwellenwertbereich seit einem bestimmten Zeitpunkt nicht durch den jeweiligen Parameter oder Messwert überschritten wurde, beträgt der Wert des Zählers 7 Null.

Wird nun ein Schwellenwert oder Schwellenwertbereich durch den aktuell erfassten Parameter oder Messwert (Ausgabe der Erfassungseinrichtung 3) verletzt (d.h. über- oder unterschritten), so wird durch die Steuereinrichtung 1 die Dauer der jeweiligen Schwellenwertverletzung gemessen und der Zähler 7 in der Speicher-/Leseeinrichtung 5 entsprechend aktualisiert.

Weiterhin kann der Zähler 7 ständig mit der entsprechenden Verifikationsintervalllänge - oder der von dieser umfassten Anzahl an Zeiteinheiten - verglichen werden. Dauert die Verletzung länger an, als das Verifikationsintervall, wird von der Steuereinrichtung 1 ein Alarmsignal generiert und mittels der Alarmeinrichtung 11 ein Alarm ausgegeben.

Verletzt der zu überwachende Parameter oder Messwert nicht mehr den Schwellenwert, wird der entsprechende Zähler 7 nicht sofort auf Null zurückgesetzt, sondern zählt rückwärts.

Eine Variante ist ein symmetrisches Dekrement, hier wird pro Zeiteinheit ohne Schwellenwertverletzung der entsprechende Zähler 7 um eine Zeiteinheit minimiert. Dies geschieht dabei vorzugsweise solange, bis der Zähler 7 entweder den Wert Null annimmt oder eine erneute Schwellenwertverletzung den Zähler 7 wieder ansteigen lässt. Durch diese Funktion kann vorteilhaft sichergestellt werden, dass auch bei um den Schwellenwert oder Schwellenwertbereich alternierenden Werten rechtzeitig ein Alarm gegeben wird.

Asymmetrische Dekremente sind ebenfalls von der Erfindung erfasst.

Fig. 2 zeigt einen Graphen zur Bestimmung automatisch generierter Schwellenwerte gemäß einer ersten Alternative des erfindungsgemäßen Verfahrens.

Der Graph zeigt den an der y-Achse aufgetragenen Verlauf 17 des zu überwachenden Parameters 13 über der auf der x-Achse 15 aufgetragenen Zeit.

Fig. 2 zeigt einen beispielhaften Werteverlauf 17 des zu überwachenden Parameters 13.

Gemäß der in Fig. 2 veranschaulichten, ersten Alternative zur Bestimmung der Schwellenwerte oder Schwellenwertbereiche, wird ein Nutzerschwellenwert 19 als niedrigster Gefährdungsgrad von einem Nutzer vorgegeben.

Anhand von Daten einer Wissensbasis wird ein parameter- und/oder patientengruppenspezifischer Notfallschwellenwert 21 bestimmt.

Die Steuereinrichtung berechnet zusätzliche mittlere Schwellenwerte oder Schwellenwertbereiche, z.B. mittels Interpolation. In Fig. 2 ist eine einzige Zwischenschwelle oder ein einziger mittlerer Schwellenwert 23 angegeben. Wie in Fig. 2 zu erkennen ist, muss der mittlere Zwischenwert 23 nicht notwendigerweise einen gemittelten Wert zwischen dem Nutzerschwellenwert 19 und dem Notfallschwellenwert 23 annehmen.

Jedem Wert oder Wertebereich wird ein spezifisches Verifikationsintervall 25 entsprechend dem mit dem Ausmaß der Abweichung des Parameters in Zusammenhang stehenden Gefährdungsgrad zugeordnet.

Wie in Fig.2 gut zu erkennen ist, weist ein Verifikationsintervall 251, welches dem Nutzerschwellenwert 19 zugeordnet ist, eine größere Länge oder Dauer auf, als ein Verifikationsintervall 253 des mittleren Schwellenwerts 23 oder gar eines Verifikationsintervalls 255 des Notfallschwellenwerts 21. Verletzt ein Parameter 13 daher den Nutzerschwellenwert 19, wird es länger dauern, bis ein Alarmsignal ausgegeben wird, als wenn der Parameter 13 den Notfallschwellenwert 21 verletzt.

Jedem Wert oder Wertbebereich ist ein Zähler 7 zugeordnet, welcher die Dauer des Zeitintervalls der Abweichung des Parameters oder Messwerts vom vorgegebenen normalen Wert oder Wertebereich erfasst. Sobald der Werteverlauf 17 des zu überwachenden Parameters 13 einen Schwellenwert übertritt, beginnt der zugehörige Zähler 7 zu zählen oder zu laufen, d.h. die Dauer der Schwellenwertverletzung in Zeiteinheiten zu erfassen.

Da in dem in Fig. 2 dargestellten Werteverlauf 17 der zu überwachende Parameter 13 den Nutzerschwellenwert 19 und den mittleren Schwellenwert 23 überschritten hat, zählen die zugehörigen Zähler 701 und 703. Da der zu überwachende Parameter 13 die Schwellen jedoch noch nicht länger als das zugehörige Verifikationsintervall 251 bzw. 253 überschritten oder verletzt hat, (schraffierte Fläche im Verifikationsintervall 251 oder 251 kleiner als die Gesamtdauer des Verifikationsintervalls) wird noch kein Alarmsignal ausgelöst.

Da der zu überwachende Parameter 13, wie in Fig. 2 gezeigt, den Notfallschwellenwert 21 (noch) nicht übertritt, ist der Zähler 705 noch auf Null gesetzt.

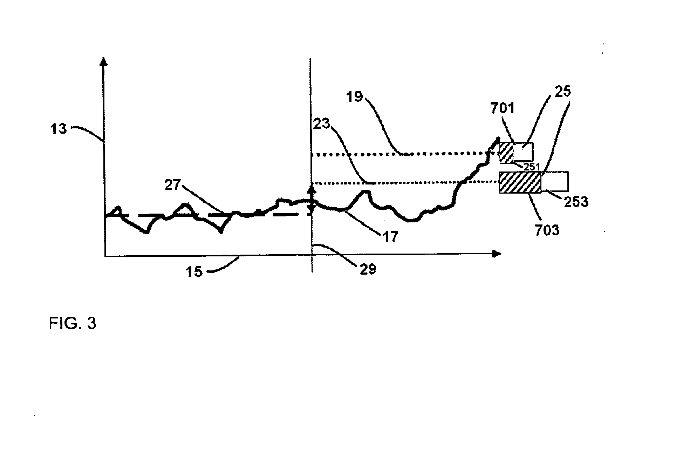

Fig. 3 zeigt einen Graphen zur Bestimmung automatisch generierter Schwellenwerte oder Schwellenwertbereiche gemäß einer zweiten Alternative.

Fig. 3 zeigt einen beispielhaften Werteverlauf 17 des zu überwachenden Parameters 13, welcher - nur zufällig - dem in Fig. 2 gezeigten Werteverlauf entspricht.

Bei der in Fig. 3 veranschaulichten, zweiten Alternative zur Bestimmung der Schwellenwerte oder Schwellenwertbereiche werden auf Basis eines von einem Nutzer eingestellten Nutzerschwellenwerts 19 und anhand von Informationen über den zu überwachenden Parameter 13, wie z.B. einem Mittelwert 27 der Parameter oder Messwerte über einen vergangenen Zeitraum, von der Steuereinrichtung automatisch patientenspezifische Schwellenwerte oder Schwellenwertbereiche bestimmt.

Sollte keine Historie zu dem zu überwachenden Parameter 13 vorliegen (in Fig.3 nicht gezeigt), könnten zur Bestimmung der Schwellenwertbereiche zunächst in der Wissensbasis hinterlegte Normwerte herangezogen werden.

Die Bestimmung der weiteren Schwellenwerte oder Schwellenwertbereiche, wie des mittleren Schwellenwertbereichs 23 kann zu einem Zeitpunkt 29 der Schwellenwertanpassung erfolgen.

In der vorliegend beschriebenen Vorgehensweise zur Schwellenwertbestimmung kann der Nutzerschwellenwert 19 dem höchsten Gefährdungsgrad entsprechen, der mittlere Schwellenwert 23 einem niedrigeren Gefährdungsgrad.

Im Folgenden wird ein Ausführungsbeispiel des erfindungsgemäßen Verfahrens zur Überwachung der Herzfrequenz (HF) hinsichtlich maximal zulässiger Werte beschrieben.

Eine Übertragbarkeit auf andere Parameter und die Überwachung hinsichtlich minimal zulässiger Werte (also unterer Grenzwerte) versteht sich von selbst. Zur Bestimmung der automatisch generierten Schwellen und für die Zuweisung der Verifikationsintervalllängen wird jeweils die vorstehend unter Bezugnahme auf Fig. 2 veranschaulichte erste Alternative zur Bestimmung automatisch generierter Schwellenwerte oder Schwellenwertbereiche verwendet.

Zur Erläuterung der einzelnen Verfahrensabläufe wird auf die vorstehend in der Zeichnung angegebenen Bezugszeichen Bezug genommen.

Im vorliegend beschrieben Ausführungsbeispiel ist ein Patient in der Ausgangssituation über EKG-Elektroden an die Erfassungseinrichtung 3 der Vorrichtung 100 zur Alarmgenerierung angeschlossen. In der Erfassungseinrichtung 3, welche als Mess- und Vorverarbeitungseinrichtung ausgestaltet sein kann, erfolgen unter anderem die Vorverarbeitung des EKG-Rohsignals und die Bestimmung der Herzfrequenz (HF).

Bei Beginn der Überwachung werden nun aus der in der Speicher-/Leseeinrichtung 5 abgelegten Wissensbasis die parameter- und/oder patientengruppenspezifischen Werte (HF) für den oberen Notfallschwellenwert 21 (beispielsweise 180/min für einen Erwachsenen) sowie die Verifikationsintervalllängen für alle drei Schwellen (Nutzerschwellenwert 19 marx. 60s, mittlerer Zwischenschwellenwert 23 max. = 15s, Notfallschwellenwert 21 max. = 4s) geladen.

Weiterhin wird der mittlere Schwellenwert 23 anhand des aktuellen Nutzerschwellenwerts 19 für HFmax wie folgt berechnet:

$$\text{Mittlerer Schwellenwert} = \text{Nutzerschwellenwert} + 0{,}3 * (\text{Notfallschwellenwert} - \text{Nutzerschwellenwert})$$

Wird beispielsweise angenommen, dass der Nutzer für HFmax einen Nutzerschwellenwert 19 von 120/min einstellt, so ergibt sich für den mittleren Schwellenwert 23 ein Wert von 138/min.

| Schwelle | Quelle | Wert | Verifikations-intervalllänge |
|---|---|---|---|
| Nutzerschwellenwert | Nutzer | 120/min | 60s |
| Mittlerer Schwellenwert | berechnet | 138/min | 15s |
| Notfallschwellenwert | Wissensbasis | 180/min | 4s |

Im Falle einer Anpassung des Nutzerschwellenwerts 19 kann der mittlere Schwellenwert 23 neu berechnet werden, wobei alle anderen zuvor erwähnten Parameter unverändert bleiben können.

Nach dieser Phase der Initialisierung überprüft die Steuereinrichtung 1 für jeden neuen Wert von HF, ob ein Schwellenwert oder Schwellenwertbereich überschritten wird oder nicht.

Steigt HF über 120/min an, wird der Zähler 701 des Nutzerschwellenwerts 19 um eine Zeiteinheit erhöht. Ist HF seit 30s größer als 120/min, so beträgt der Wert des Zählers 701 folglich 30s.

Bei jeder Veränderung eines Zählers 7 wird überprüft, ob der Zählerwert größer ist als die dazugehörige Verifikationsintervalllänge. Ist dies der Fall, wird durch die Alarmeinrichtung 11 ein entsprechendes Alarmsignal ausgegeben.

Fällt der Wert für HF beispielsweise wieder unter 120/min ab, werden die Zähler 7 für jede Zeiteinheit unter den jeweiligen Schwellenwert oder Schwellenwertbereich verringert. Hat HF also für 10s den Wert 100/min, verringert sich der Wert des Zählers 701 des Nutzerschwellenwerts 19 wieder auf 20s.

Steigt nun der HF erneut an, werden die Zähler 7 wieder entsprechend erhöht.

Steigt HF beispielsweise plötzlich von 100/min auf 185/min an, werden die Zähler 7 für alle Schwellenwerte oder Schwellenwertbereiche (Nutzerschwellenwert 19 / mittlerer Schwellenwert 23 / Notfallschwellenwert 21) hoch gezählt, da der Wert alle Schwellenwerte verletzt.

Beträgt die HF nun länger als 4s 185/min, so ist der Wert des Zählers 705 des Notfallschwellenwerts 21 größer als die zugehörige Verifikationsintervalllänge - es wird ein Alarmsignal ausgegeben. Zu diesem Zeitpunkt beträgt der Stand des Zählers 701 für den Nutzerschwellenwert 19 25s, der Stand des Zählers 703 für den mittleren Schwellenwert 23 beträgt 5s. Nach Bestätigung des Alarms durch den Nutzer werden alle Zähler 7 auf 0s zurückgesetzt.

BEZUGSZEICHENLISTE

100    Vorrichtung zur Alarmgenerierung
1      Steuereinrichtung
3      Erfassungseinrichtung
5      Speicher-/Leseeinrichtung
7      Zähler
701    Zähler des Nutzerschwellenwerts
703    Zähler des mittleren Schwellenwerts
705    Zähler des Notfallschwellenwerts
9      Benutzerschnittstelle
901    Eingabeeinrichtung
903    Ausgabeeinrichtung
11     Alarmeinrichtung
12     Datenleitungen
13     Parameter
15     Zeit
17     Werteverlauf
19     Nutzerschwellenwert
21     Notfallschwellenwert
23     Mittlerer Schwellenwert
25     Verifikationsintervall
27     Mittelwert der Parameter/Messwerte
29     Zeitpunkt der Schwellenwertanpassung
251    Verifikationsintervall des Nutzerschwellenwerts

253 Verifikationsintervall des mittlerer Schwellenwerts
255 Verifikationsintervall des Notfallschwellenwerts

**Patentansprüche**

1. Verfahren zur Alarmgenerierung mit den Schritten:

   Erfassen oder Bestimmen eines Parameters (13) im Falle seines Abweichens von einem vorgegebenen Wert oder Wertebereich unter Betrachtung eines zeitlich begrenzten Verifikationsintervalls (25),
   Anpassen der zeitlichen Begrenzung des Verifikationsintervalls (25) in Abhängigkeit vom Ausmaß des Abweichens des Parameters (13) von einem vorgegebenen Wert oder Wertebereich,
   Generieren eines Alarms, falls der überwachte Parameter (13) über das ganze Verifikationsintervall von dem vorgegebenen Wert oder Wertebereich abweicht.

2. Verfahren nach Anspruch 1, mit dem weiteren Schritt:

   Festlegen eines ersten oberen, oder unteren, Schwellenwerts oder Schwellenwertbereichs, bei oder mit dessen Überschreiten, oder Unterschreiten, ein Beginn eines Verifikationsintervalls einer ersten Zeitdauer definiert wird; und/oder Festlegen eines zweiten, ebenfalls oberen, oder unteren, Schwellenwerts oder Schwellenwertbereichs, bei oder mit dessen Überschreiten, oder Unterschreiten, ein Beginn eines Verifikationsintervalls einer zweiten Zeitdauer definiert wird, wobei die zweite Zeitdauer von der ersten Zeitdauer verschieden ist.

3. Verfahren nach Anspruch 1 oder 2, wobei wenigstens ein Schwellenwert (23) oder Schwellenwertbereich anhand von Daten einer vor Beginn des Überwachungsverfahrens bekannten Wissensbasis bestimmt wird und/oder basierend auf wenigstens einem im laufenden Überwachungsverfahren ermittelten Wertes automatisch festgelegt wird.

4. Verfahren nach einem der vorangegangenen Ansprüche, wobei wenigstens ein Schwellenwert (23) oder Schwellenwertbereiche anhand von Daten einer vor Beginn des Überwachungsverfahrens bekannten Wissensbasis bestimmt wird und/oder basierend auf wenigstens einem im laufenden Überwachungsverfahren ermittelten Werten automatisch festgelegt wird.

5. Verfahren nach einem der vorangegangenen Ansprüche, wobei es sich bei den überwachten Werten um Vitalparameter eines Patienten handelt.

6. Verfahren nach einem der vorangegangenen Ansprüche, wobei ein Zähler (7) dekrementiert wird, wenn der Parameter (13) eine Zeiteinheit lang auf einen Wert unterhalb eines für den Zähler (7) relevanten Schwellenwertes (19, 21, 23) absinkt und der Wert des Zählers größer Null ist.

7. Steuereinrichtung (1), welche Mittel hat um ein Verfahren nach einem der Ansprüche 1 bis 6 auszuführen.

8. Vorrichtung (100) zur Alarmgenerierung, falls wenigstens ein überwachter Parameter (13) eines Patienten von vorgegebenen normalen Werten oder Wertebereichen abweicht, mit:

   wenigstens einer Steuereinrichtung (1) gemäß Anspruch 7, wenigstens einer Erfassungseinrichtung (3) zum Erfassen oder Bestimmen eines Parameters (13),
   wenigstens einer Vergleichseinrichtung zum Vergleichen des erfassten Parameters (13) mit vorgegebenen Werten oder Wertebereichen, und vorzugsweise wenigstens einer Alarmeinrichtung (11).

9. Vorrichtung (100) nach Anspruch 8, ferner aufweisend:

   wenigstens eine Speichereinrichtung oder Speicher/Leseeinrichtung (5) zum Abspeichern oder Hinterlegen von Ergebnissen.

10. Vorrichtung (100) nach einem der Ansprüche 8 oder 9, ferner aufweisend:

   wenigstens eine Eingabeeinrichtung (901) zum Eingeben der vorbekannten Werte oder Wertebereiche.

**11.** Vorrichtung (100) nach einem der Ansprüche 8 bis 10, ferner aufweisend:

wenigstens eine Ausgabeeinrichtung (903) zum Ausgeben von Ergebnissen der Steuereinrichtung.

**12.** Behandlungsvorrichtung zum Überwachen eines Zustands eines Patienten, welche wenigstens eine Steuereinrichtung (1) gemäß Anspruch 7 oder wenigstens eine Vorrichtung (100) zur Alarmgenerierung gemäß einem der Ansprüche 8 bis 11 aufweist.

**13.** Digitales Speichermedium, insbesondere Diskette, CD oder DVD, mit elektrisch auslesbaren Steuersignalen, die mit einem programmierbaren Computersystem zusammenwirken, dass ein Verfahren nach einem der Ansprüche 1 bis 6 ausgeführt wird.

**14.** Computer-Programm mit Programmcode zur Durchführung des Verfahrens nach einem der Ansprüche 1 bis 6, wenn das Programm auf einem Computer abläuft.

**15.** Computer-Programm-Produkt mit einem auf einem maschinenlesbaren Träger gespeicherten Computer-Programm nach Anspruch 14.

**Claims**

**1.** A method for generating an alarm comprising the steps:

detecting or determining a parameter (13) in case of a deviation of the parameter from a preset value or value range taking into consideration a verification interval (25) of limited duration in time,
adapting the time limitation of the verification interval (25) as a function of the extent of the deviation of the parameter (13) from the preset value or value range,
generating an alarm if the monitored parameter (13) deviates from the preset value or value range over the entire verification interval.

**2.** A method according to claim 1, comprising the further step:

setting a first upper or lower threshold value or a first threshold value range, wherein, upon overshooting or undershooting the set first upper or lower threshold value or first threshold value range, the beginning of a verification interval with a first duration is defined; and/or setting a second upper or lower threshold value or a second threshold value range, wherein, upon overshooting or undershooting the second upper or lower threshold value or second threshold value range, the beginning of a verification interval of a second duration is defined, wherein the second duration is different from the first duration.

**3.** A method according to claim 1 or 2, wherein at least one threshold value (23) or threshold value range is determined on the basis of data of a knowledge base known before the beginning of the monitoring process and/or is set automatically on the basis of at least one value determined during the ongoing monitoring process.

**4.** A method according to any of the preceding claims, wherein at least one threshold value (23) or threshold value range is determined on the basis of data of a knowledge base known before the beginning of the monitoring process and/or set automatically on the basis of at least one value determined during the ongoing monitoring process.

**5.** A method according to any of the preceding claims, wherein said at least one monitored parameter is a vital parameter of a patient.

**6.** A method according to any of the preceding claims, wherein a counter (7) is decremented if the parameter (13) drops to a value below a threshold value (19, 21, 23) relevant for the counter (7) for a length of a unit of time and the value of the counter is greater than zero.

**7.** A control device (1) which comprises means for carrying out a method according to any of the claims 1 to 6.

**8.** A device (100) for generating an alarm if at least one monitored parameter (13) of a patient deviates from preset normal values or value ranges, comprising:

at least one control device (1) according to claim 7;
at least a detecting means (3) for detecting or determining a parameter (13);
at least one comparison means for comparing the detected parameter (13) with preset values or value ranges, and preferably at least one alarm device (11).

9. A device (100) according to claim 8, further comprising:

at least one memory means or memory/reading means (5) for storing or saving results.

10. A device (100) according to one of claims 8 or 9, further comprising:

at least one input means (901) for inputting previously known values or value ranges.

11. A device (100) according to one of the claims 8 to 10, further comprising:

at least one output means (903) for outputting results of the control device.

12. A treatment device for monitoring a state of a patient, the treatment device comprising at least one control device (1) according to claim 7 or at least one device (100) for generating an alarm according to one of the claims 8 to 11.

13. A digital storage medium, in particular a diskette, CD or DVD, comprising electrically readable control signals which cooperate with a programmable computer system to carry out a method according to one of the claims 1 to 6.

14. A computer program with program code for carrying out a method according to one of the claims 1 to 6 when the program is executed on a computer.

15. A computer program product comprising a computer program according to claim 14 stored on a machine readable carrier.

**Revendications**

1. Procédé de génération d'alarme, comportant les étapes suivantes :

détection ou détermination d'un paramètre (13) dans le cas où celui-ci s'écarte d'une valeur ou d'une plage de valeurs prédéfinie, en prenant en considération un intervalle de vérification (25) limité dans le temps,
adaptation de la limitation dans le temps de l'intervalle de vérification (25) en fonction de l'étendue de l'écart du paramètre (13) d'une valeur ou d'une plage de valeurs prédéfinie,
génération d'une alarme si le paramètre surveillé (13) s'écarte de la valeur ou de la plage de valeurs prédéfinie sur tout l'intervalle de vérification.

2. Procédé selon la revendication 1, comportant l'étape supplémentaire suivante :

définition d'une première valeur seuil ou plage de valeurs seuils supérieure ou inférieure, dont le surpassement ou le soupassement définit le début d'un intervalle de vérification d'une première durée ; et/ou définition d'une deuxième valeur seuil ou plage de valeurs seuils, également supérieure ou inférieure, dont le surpassement ou le soupassement définit le début d'un intervalle de vérification d'une deuxième durée, la deuxième durée étant différente de la première durée.

3. Procédé selon la revendication 1 ou 2, dans lequel au moins une valeur seuil (23) ou une plage de valeurs seuils est déterminée à partir de données d'une base de connaissances connue avant le début du procédé de surveillance et/ou est définie automatiquement sur la base d'au moins une valeur déterminée dans le procédé de surveillance en cours.

4. Procédé selon une des revendications précédentes, dans lequel au moins une valeur seuil (23) ou une plage de valeurs seuils est déterminée à partir de données d'une base de connaissances connue avant le début du procédé de surveillance et/ou est définie automatiquement sur la base d'au moins une valeur déterminée dans le procédé de surveillance en cours.

**5.** Procédé selon une des revendications précédentes, dans lequel les valeurs surveillées sont des paramètres vitaux d'un patient.

**6.** Procédé selon une des revendications précédentes, dans lequel un compteur (7) est décrémenté lorsque le paramètre (13) tombe, pendant une unité de temps, à une valeur inférieure à une valeur seuil (19, 21, 23) pertinente pour le compteur (7) et lorsque la valeur du compteur est supérieure à zéro.

**7.** Dispositif de commande (1) qui comporte des moyens pour réaliser un procédé selon une des revendications 1 à 6.

**8.** Dispositif (100) de génération d'alarme si au moins un paramètre surveillé (13) d'un patient s'écarte de valeurs ou de plages de valeurs normales prédéfinies, comprenant :

au moins un dispositif de commande (1) selon la revendication 7,
au moins un dispositif de détection (3) pour détecter ou déterminer un paramètre (13),
au moins un dispositif de comparaison pour comparer le paramètre (13) détecté avec des valeurs ou des plages de valeurs prédéfinies, et, de préférence, au moins un dispositif d'alarme (11).

**9.** Dispositif (100) selon la revendication 8, comportant en outre :

au moins un dispositif de mémoire ou un dispositif de mémoire/lecture (5) pour mémoriser ou stocker des résultats.

**10.** Dispositif (100) selon une des revendications 8 ou 9, comportant en outre :

au moins un dispositif d'entrée (901) pour entrer les valeurs ou plages de valeurs déjà connues.

**11.** Dispositif (100) selon une des revendications 8 à 10, comportant en outre :

au moins un dispositif de sortie (903) pour sortir les résultats du dispositif de commande.

**12.** Dispositif de traitement pour surveiller un état d'un patient, lequel comporte au moins un dispositif de commande (1) selon la revendication 7 ou au moins un dispositif (100) de génération d'alarme selon une des revendications 8 à 11.

**13.** Support de stockage numérique, en particulier disquette, CD ou DVD, avec des signaux de commande lisibles électriquement qui coopèrent avec un système informatique programmable de manière à réaliser un procédé selon une des revendications 1 à 6.

**14.** Programme informatique avec un code de programme pour réaliser le procédé selon une des revendications 1 à 6 lorsque le programme s'exécute sur un ordinateur.

**15.** Programme informatique avec un programme d'ordinateur selon la revendication 14 stocké sur un support lisible par un ordinateur.

FIG. 1

FIG. 2

FIG. 3

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- US 20080208026 A1